# EUROPEAN PATENT APPLICATION

(11) **EP 3 427 760 A1**
(43) Date of publication of application: **16.01.2019**
(21) Application number: 18188360.4
(22) Date of filing: 11.06.2015
(51) Int. Cl.: A61L 2/20, D06M 11/64

(54) **A METHOD FOR DISINFECTING CLOTHES, SHOES OR EQUIPMENT USING NITRIC OXIDE**

(30) Priority: 13.06.2014 US 201462011844 P
(62) Divisional of application: 15806903.9
(71) Applicant: Origin, Inc., Princeton, NJ 08540 (US)
(72) Inventor: Nelson, Howard, Pennington, NJ 08534 (US); Dolgopolsky, Alexander, Richboro, PA 18954 (US); Vasilets, Victor, 142432 Chernogolovka (RU); Kocsardy, Jennifer, Pennington, NJ 08534 (US)
(74) Representative: Murgitroyd & Company

(57) **Abstract**

A method for disinfecting clothes, shoes or equipment using nitric oxide is provided. The method comprises creating a discrete stream of matter that includes nitric oxide (NO) in a concentration from about 5 ppm to 3500 ppm, and directing the discrete stream of matter at a surface to disinfect the surface.

## Description

### Field of the Disclosure

The disclosure relates to methods for effectively administering nitric oxide in the disinfection of clothes, shoes or equipment..

### Background of the Disclosure

Nitric Oxide (NO) gas is a short-lived molecule normally found in a gaseous state both inside and outside the animal body. NO is a signaling molecule known to have numerous regulatory, protective and therapeutic properties. Augmenting an animal's natural generation of NO by either stimulating increased production of endogenous NO or introducing exogenously-produced NO into the animal can improve the animal's response to damage, pain, and invading organisms.

### Summary

It would be advantageous to provide a method for administering NO to clothes, shoes and equipment in a manner that facilitates disinfection of those items..

In accordance with the present disclosure, there is provided a method for disinfecting clothes, shoes or equipment using nitric oxide, the method comprising:
creating a discrete stream of matter that includes nitric oxide (NO) in a concentration from about 5 ppm to 3500 ppm; and
directing the discrete stream of matter at a surface to disinfect the surface.

### Brief Description of the Drawings

**FIG. 1** illustrates a first exemplary device for producing NO according to the disclosure;
**FIG. 2** illustrates a second exemplary device for producing NO according to the disclosure;
**FIG. 3** illustrates a third exemplary device for producing NO according to the disclosure; and
**FIG. 4** is a flow diagram illustrating a first exemplary method in accordance with an embodiment of the present invention.

### Detailed Description

The present invention will now be described more fully hereinafter with reference to the accompanying drawings, in which preferred embodiments of the invention are shown. The invention, however, may be embodied in many different forms and should not be construed as being limited to the embodiments set forth herein. Rather, these embodiments are provided so that this disclosure will be thorough and complete, and will fully convey the scope of the invention to those skilled in the art. In the drawings, like numbers refer to like elements throughout.

In accordance with the present disclosure, a method is presented for creating a discrete stream of matter in a plasma state, where the stream has as part of its content NO, and administering such stream to clothes, shoes or equipment for the disinfection thereof.

In some embodiments, NO application at the surface level (i.e., directed at the skin or open wound) is believed to stimulate the animal's own production of NO such that therapeutic effects can be obtained at and around the indication site. Alternatively, the disclosed methods may exploit the fact that NO in the plasma state is of sufficiently high energy and velocity that it can penetrate through and around cellular membranes. In some cases the NO may pass through biofilms and the stratum corneum to produce therapeutic results in the associated animal tissue.

Matter in a plasma state that contains NO can be created via several methods. Atmosphere contains nitrogen and oxygen, and thus, sufficient energy in the correct geometry can produce nitric oxide from the gaseous mixture. Energy can be added to transition the gaseous N₂ and O₂ into the plasma state. In one non-limiting, exemplary embodiment, pre-formed gaseous in N₂-O₂ mixture can be created and passed through a plasma energy arc that transfers sufficient energy to production of NO in a plasma state.

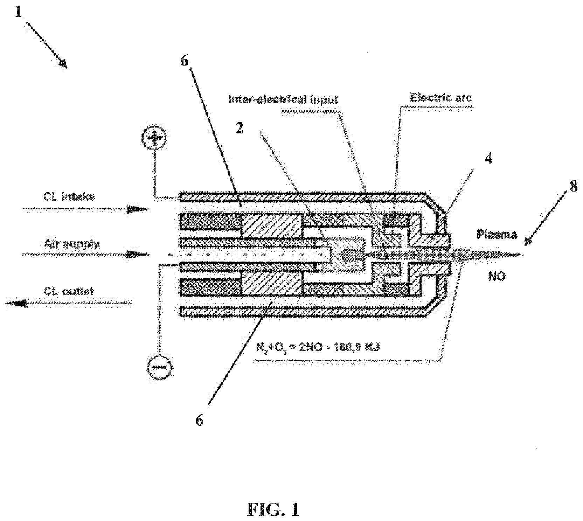

**FIG. 1** shows an exemplary generator portion of a NO production device 1 for use in carrying out one or more of the disclosed methods. As can be seen, air is introduced at a first end of the device 1, and is channeled between a pair of electrodes, namely a cathode 2 and an anode 4 which are insulated from each other. A stationary DC arc discharge is generated and maintained between the electrodes 2, 4. A NO-containing gas flow is formed from the air in the area between the electrodes 2, 4 under the effect of the arc discharge, and is withdrawn through a cooled channel (cooled by a coolant loop 6), enabling NO to be fixed in the flow 8. The temperature of the flow and the NO content can be brought to desired values for providing a therapeutic benefit to a treatment site. Further details regarding the illustrated exemplary NO production device 1 can be found, for example, in U.S. Patent No. 7,498,000 to Pekshev, the entirety of which is incorporated herein by reference.

Classical thermodynamics confirmed by compositional analysis of the thermodynamical equilibrium of air in a plasma state shows that at a temperature lower than 2000°C the concentration of NO in the gas does not exceed 1%. Increasing the plasma temperature increases the NO concentration up to its maximum (∼5%) at a temperature of 3500-4000°C. Slightly less than 4000°C is the temperature of the electrical discharge in the plasma arc of the illustrated device 1. Plasma-chemical reactions, which lead to the formation NO, can be expressed by the following chemical formula:

N₂ + O₂ → 2NO - 180.9 kilo-Joules per mol (kJ/mol)

Life-time of the NO molecule at high temperatures is comparable to the time of its synthesis because of fast reaction of recombination (2NO + O₂ = 2NO₂)+. To prevent recombination and keep therapeutic concentration of NO for medical applications, it is desirable to accomplish rapid cooling of the reaction mixture, i.e., quenching. Quenching of the NO occurs with braking of the escaping flow in surrounding cold air (i.e., from coolant loop 6). The illustrated device 1 enables a direct current plasma arc to be created using ambient air at atmospheric pressure. The device 1 produces a stream of hot air 8 with a composition of plasma species that contains medically significant amount of NO, which in one exemplary embodiment is about 2,500 parts per million (ppm) NO.

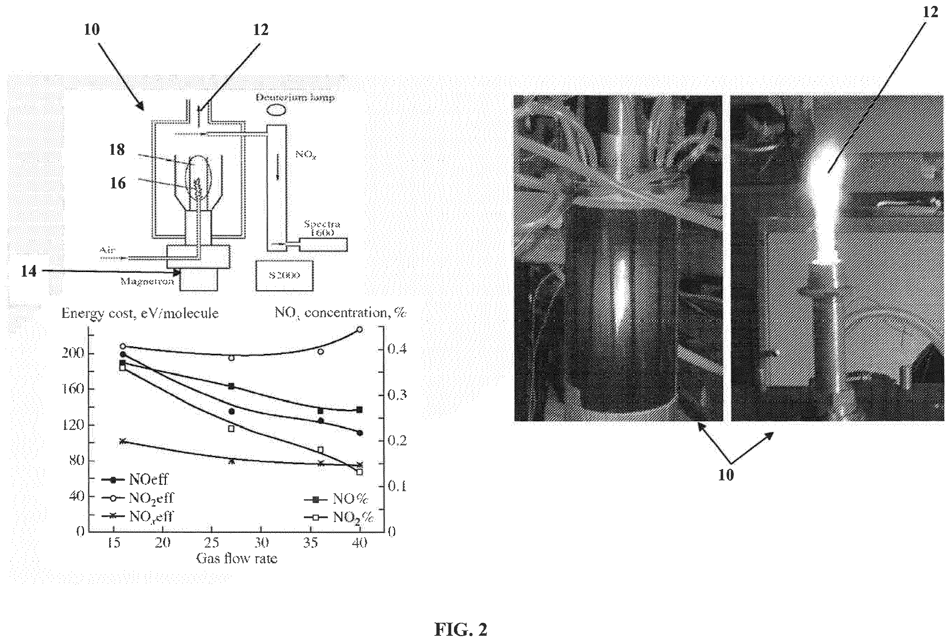

The device 1 shown in **FIG. 1** is not exclusive, and alternative sources of plasma-generated NO may also be used to carrying out one or more of the disclosed methods. **FIG. 2** shows such an alternative device 10 for production of NO-containing matter in a plasma state 12 for use in carrying out one or more of the disclosed methods. This device 10 employs microwave discharge technology for producing matter in a plasma state 12 having a desired composition (i.e., about 2,000 ppm of NO). The illustrated device 10 includes a magnetron 14 having a power of P < 1 kilowatt (kW) and a frequency of 2.45 gigahertz (GHz). Air is passed by the magnetron 14 and directed to a core portion 16 of the torch body 18, where a stream of matter in a plasma state 12 is generated and output for application to a targeted treatment site.

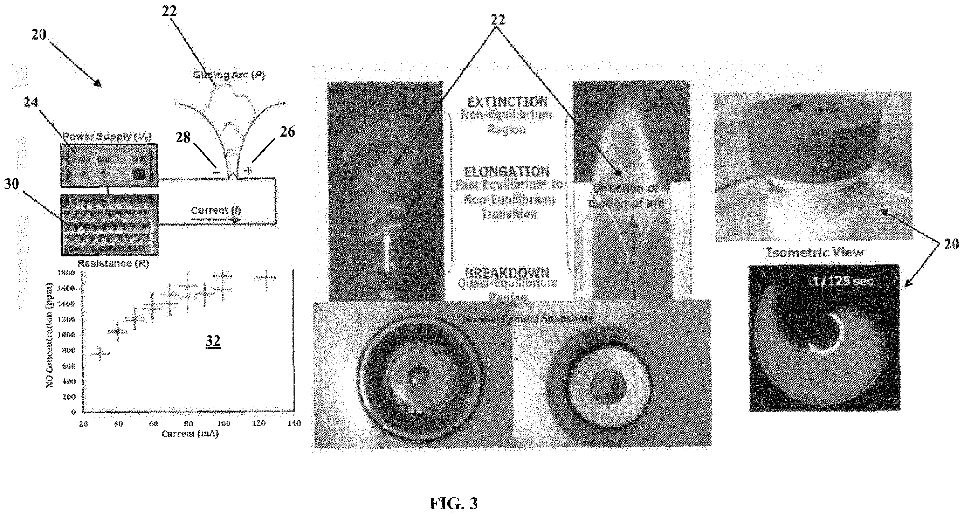

**FIG. 3** shows yet another device 20 for production of NO-containing matter in a plasma state for use in carrying out one or more of the disclosed methods. The illustrated device 20 employs magnetically stabilized gliding arc discharge technology for producing matter in a plasma state 22 having a desired composition (again, about 2,000 ppm of NO).

A gliding arc is operated in air at atmospheric pressure, but at moderate power levels (typically between 50 and 300 Watts). A power source 24 and anode/cathode 26, 28 is employed, and current is restricted using an external ballast resistor 30. This heats the discharge (i.e., the plasma jet 22) to moderate temperatures (2000-3000 degrees Kelvin), while preserving non-equilibrium nature of the discharge (Te>Tg). As such, higher concentrations of NO (e.g., 1600-1800 ppm) can be obtained at lower power input. A graph 32 shows the relationship between NO concentration (ppm) of the matter in a plasma state 22 vs. discharge current (mA).

As noted, the described devices shown in **FIGS. 1-3** are not exclusive, and alternative sources of plasma-generated NO may also be used to carrying out one or more of the disclosed methods. Such alternative devices may produce matter in a plasma state having the desired compositions up to, and/or exceeding, 3,500 ppm NO.

As will be appreciated, NO in a plasma state can be used for a variety of purposes. For example, NO in the plasma state can be used as an antimicrobial agent. In addition, NO in the plasma state can be used to reduce inflammation, or to facilitate vasodilation. NO in the plasma state further can be employed to alleviate pain associated with osteoarthritis and Rheumatoid Arthritis. It can also be effective in combating Gram Positive microorganisms, Gram Negative microorganisms, Fungi (including causes of onychomycosis- trichophyton rubrum, candida and mold scytalidium) and viruses. It is also therapeutic in treating osteoporosis, collagen formation, stem cell signaling, satellite cell differentiation, wound-healing, wound-management, reduction in scar tissue, and remediation of activity related injury. NO in a plasma state can also aid in nerve regeneration, can inhibit cancer cell proliferation, can promote apoptosis, can stimulate endogenous nitric oxide production, and can stimulate iNOS pathways.

In practice, the NO in a plasma state can be applied directly to or adjacent to living animal tissue in order to produce a desired effect. It can effectively function to maintain homeostasis in the cardiovascular and respiratory systems. NO, as a signaling molecule, can cause vasodilation which promotes blood vessel flexibility, eases blood pressure, cleans the blood, reverses atherosclerosis and effectively prevents cardiovascular diseases and aids in its recovery. Another important function of NO is slowing down atherosclerotic plaque deposition on vascular walls. NO also plays an active defense role in the immune system. It is a strong antioxidant, and can suppress bacterial infections, viruses and parasitic attacks. It can even deter some types of cancer cell growth. In patients with moderate to severe diabetes, NO can prevent many common and serious complications. NO can also significantly reduce the pain associated with joint swelling in arthritis. NO can effectively decrease the risk of cancer, diabetes, myocardial infarction and stroke.

In the nervous and endocrine systems, NO can induce normal functioning of various body organs. NO can permeate freely through the cell membrane for biological signaling, adjust cellular activities and lead every organ to complete its function properly, including the lungs, liver, kidneys, stomach, heart, brain and genitals. NO can increase blood flow to the genital organs to maintain normal sexual function. The brain transmits signals via its surrounding nerves to the perineal region to provide it with sufficient NO to cause vascular dilation, increasing blood flow to enhance erectile function. Under some conditions, weak erections are the results of insufficient NO production by nerve endings.

The NO molecules produced by the immune system are not only capable of destroying invading microorganisms, but also help activate and nourish brain cells, significantly slowing aging and improving memory.

### EXEMPLARY CONDITIONS

A non-limiting listing of exemplary conditions for which the disclosed NO-containing matter in a plasma state may find beneficial use as a veterinary treatment includes:

### Preventative Applications

Infection Prevention, Including
Open Wounds
Bites
Punctures
Pre and Post-Surgical Sites
Castration
Docking
Ear Cropping
Foot Trimming
Microchip Installation

### Skin/Soft Tissue/Nail Infections

Emerging Diseases Involving Bacterial/Fungal Surface Infections
Fungal Infections (Surface/Skin) - e.g., Mange, Ringworm
Gum Line Infections
Hoof Bacterial Infections - e.g., Thrush, Foot Abscesses
Infectious Foot Rot - e.g., Interdigital Pododermatitis
Infection Due to Disbudding and Dehorning
Mastitis
Skin Infections, Allergies
Skin Irritations - e.g., Due to Fly/Flea/Tick Bites and Stinging Insects, Animal Bites/Scratches
Strangles Related Issues - e.g., Abscessed Lymph Nodes
Toe Nail Infections
Tusk Trimming Infections

### Trauma/Wounds/Inflammation

Arthritis/Polyarthritis
Edema - general
Joint inflammation - e.g., joint edema
Lameness
Muscle Inflammation/Injury
Soft Tissue Trauma - e.g., bruise or contusion

### Equipment Disinfection

Mechanical Transmission of Bacteria/Infection (Carried on Clothes, Shoes or Equipment)
Avoidance of Transmission from Farm to Farm

### EXEMPLARY PATHOGENS

A non-limiting listing of exemplary pathogens for which the disclosed NO-containing matter in a plasma state may find beneficial use as a veterinary treatment includes:
Bacillus Necrophorus
Bacteriodes melaninogenicus
Bacteriodes nodosus
Bacteroides fragilis
Bacteroides splanchnicus
Clostridium septicum
C. chauvoei
C. novyi
C. perfringens
C. septicum
C. sordellii
C. tetani
Campylobacter jejuni
Demodex (live in follicles)
Escherichia coli
Follivulitis
Fusobacterium necrophorum
Fusobacterium necrophorum
Gingivitis
Impetigo
Lactobacillus plantarum
Onychomycosis
Paronychia
Peptostreptococcus
Peridontitis
Porphyromonas denticanis
Porphyromonas gulae
Porphyromonas salivosa
Prevotella intermedia
Salmonella enterica
Sarcoptes (burrows in skin)
Staphylococcus aureus
Staphylococcus hyicus
Staphylococcus spp and Streptococcus spp
Streptococcus agalactiae
Streptococcus equi
Streptococcus pyogenes
Tinea Corporsis
Treponema spp

An exemplary baseline composition of matter in a plasma state is shown in **Table I** below. It will be appreciated that this composition itemization is merely exemplary, and that other compositions can also be used to beneficial effect.

**TABLE I**

| **BASELINE PLASMA COMPOSITION** | | |
|---|---|---|
| **Parameter** | **Minimum Value** | **Maximum Value** |
| Nitric Oxide (NO) | 5 ppm | 3500 ppm |
| NO₂ | 0 ppm | 200 ppm |
| N₂ | 75 vol% | 78 vol% |
| O₂ | 18 vol% | 21 vol% |
| O₃ | 0 ppm | 0.1 ppm |
| H₂O₂ | 0 ppm | 1500 ppm |
| H₂O | 0 ppm | 20000 ppm |
| Ar | 0 ppm | 9.1 * 10^5 ppm |
| He | 5.2 ppm | 9.1 * 10^5 ppm |
| CO | 0 ppm | 50 ppm |
| CO₂ | 0 ppm | 500 ppm |
| H₂ | 0 ppm | 10000 ppm |

An exemplary baseline treatment scheme is shown in **Table II** below. It will be appreciated that this baseline scheme may be adjusted, as will be described in relation to a number of Examples to follow, to provide a desired treatment plan for an affected area and in response to a particular indication.

As shown in **Table II,** the treatment variables include "distance from exit to site," "time of application," "number of treatments," "length of time between treatments," "temperature of plasma stream at contact with site," and "velocity of plasma stream at contact with treatment site."

"Distance from exit to site" will be understood to be the standoff distance, in centimeters, from the outlet of the plasma device (e.g., device 1, 10, 20) to the treatment site. "Time of application" will be understood to be the amount of time, in seconds, that the NO-containing matter in a plasma state will be directed from the plasma device onto the treatment site, per square centimeter of site area. Thus, the time of application will depend upon the size of the area being treated. "Number of treatments" will be understood to be the discrete number of treatments to be applied at the site. "Length of time between treatments" will be understood to be the amount of time elapsed between applications of the NO-containing matter in a plasma state at the treatment site. "Temperature of plasma stream at contact with treatment site" will be understood to be the temperature of the NO-containing matter in a plasma state, in degrees Celsius, at the treatment site. "Velocity of plasma stream at contact with treatment site" will be understood to be the speed of the NO-containing matter in a plasma state, in meters per second, at the treatment site. Minimum and maximum values are provided for each, recognizing that individual treatment specifications for particular indications will vary within the indicated ranges.

**TABLE II**

| **BASELINE TREATMENT SCHEME** | | |
|---|---|---|
| **Parameter** | **Minimum Value** | **Maximum Value** |
| Distance from exit to site (cm) | 1 | 25 |
| Time of Application (sec/cm²) | 5 | 45 |
| Number of treatments | 1 | 24 |
| Length of time between treatments (hours) | 3 | 168 |
| Temperature of plasma stream at contact with treatment site °C | 10 | 60 |
| Velocity of Plasma Stream at contact with treatment site (m/sec) | 0.05 | 200 |

A series of exemplary specific treatment schemes will now be discussed in relation to various indications. These treatment schemes assume use of matter in a plasma state having the compositions identified in **Table I.**

### EXAMPLE 1

### Treatment Scheme - Preventative Applications

The previously-identified "Preventative Applications," can be treated according to the parameters identified in **Table 1** below. The minimum treatment values and maximum treatment values are based on reducing bacterial load in tissue. Minimum treatment parameters define the requirements for the initiation of the decolonization process. Application of therapy can increase in intensity, duration and frequency as the severity increases.

### Example 1 - Table 1

| **Parameter** | **Minimum Value** | **Maximum Value** |
|---|---|---|
| Distance from exit to site (cm) | 1 | 50 |
| Time of Application (sec/cm²) | 5 | 45 |
| Number of treatments | 1 | 10 |
| Length of time between treatments (hours) | 3 | 168 |
| Temperature of plasma stream at contact with Site °C | 10 | 60 |
| Velocity of Plasma Stream at contact with Site (m/sec) | 0.05 | 200 |

### EXAMPLE 2

### Treatment Scheme - Inflammation

Inflammation caused by any of the previously-identified bacteria or other conditions can be treated according to the parameters identified in **Table 2** below. The minimum treatment values and maximum treatment values are based on severity of inflammation, mobility and pain. Length of time is variable due to the depth of the joint beneath the surface of the skin and the amount of surrounding soft tissue. Severity is determined by the level of inflammation, mobility and pain symptoms. Application of therapy increases in intensity, duration and frequency as the severity increases. Minimum treatment parameters define the requirements for the initiation of the inflammation care management process. Treatment includes a border around the site of up to 1 cm due to circulatory issues.

### Example 2 - Table 2

| **Parameter** | **Minimum Value** | **Maximum Value** |
|---|---|---|
| Distance from exit to site (cm) | 1 | 25 |
| Time of Application (sec/cm²) | 15 | 120 |
| Number of treatments | 3 | 50 |
| Length of time between treatments (hours) | 12 | 168 |
| Temperature of plasma stream at contact with Site °C | 10 | 60 |
| Velocity of Plasma Stream at contact with Site (m/sec) | 0.05 | 200 |

### EXAMPLE 3

### Treatment Scheme - Wounds

Wounds, such as, but not limited to, the previously identified listing, can be treated according to the parameters identified in **Table 3** below. The minimum treatment values and maximum treatment values are based on severity and size of wounds. Severity of the wound is determined by the surface area, depth, and symptoms. Application of therapy increases in intensity, duration and frequency as the severity increases. Minimum treatment parameters define the requirements for the initiation of the wound care management process.

### Example 3 - Table 3

| **Parameter** | **Minimum Value** | **Maximum Value** |
|---|---|---|
| Distance from exit to site (cm) | 1 | 45 |
| Time of Application (sec/cm²) | 1 | 45 |
| Number of treatments | 1 | 200 |
| Length of time between treatments (hours) | 3 | 168 |
| Temperature of plasma stream at contact with Site °C | 10 | 60 |
| Velocity of Plasma Stream at contact with Site (m/sec) | 0.05 | 200 |

### EXAMPLE 4

### Treatment Scheme - Gram Negative Bacteria

Gram negative bacteria, such as, but not limited to, the previously-identified listing, can be treated according to the parameters identified in **Table 4** below. The minimum treatment values and maximum treatment values are based on severity of the gram negative bacterial infection. Severity of the infection is determined by the surface area, depth, colony count and symptoms. Application of therapy increases in intensity, duration and frequency as the severity increases. Minimum treatment parameters define the requirements for the initiation of the decolonization process. Gram negative bacteria are more difficult to kill than gram positive, so longer treatments are required to decolonize.

### Example 4 - Table 4

| **Parameter** | **Minimum Value** | **Maximum Value** |
|---|---|---|
| Distance from exit to site (cm) | 1 | 45 |
| Time of Application (sec/cm²) | 10 | 90 |
| Number of treatments | 1 | 24 |
| Length of time between treatments (hours) | 3 | 168 |
| Temperature of plasma stream at contact with Site °C | 10 | 60 |
| Velocity of Plasma Stream at contact with Site (m/sec) | 0.05 | 200 |

### EXAMPLE 5

### Treatment Scheme - Gram Positive Bacteria

Gram positive bacteria, such as, but not limited to, the previously-identified listing, can be treated according to the parameters identified in **Table 5** below. The minimum treatment values and maximum treatment values are based on severity of the gram positive bacterial infection. Severity of the infection is determined by the surface area, depth, colony count and symptoms. Application of therapy increases in intensity, duration and frequency as the severity increases. Minimum treatment parameters define the requirements for the initiation of the decolonization process.

### Example 5 - Table 5

| **Parameter** | **Minimum Value** | **Maximum Value** |
|---|---|---|
| Distance from exit to site (cm) | 1 | 45 |
| Time of Application (sec/cm²) | 5 | 45 |
| Number of treatments | 1 | 24 |
| Length of time between treatments (hours) | 3 | 168 |
| Temperature of plasma stream at contact with Site °C | 10 | 60 |
| Velocity of Plasma Stream at contact with Site (m/sec) | 0.05 | 200 |

### EXAMPLE 6

### Treatment Scheme - Fungus

Fungus, including, but not limited to, the previously-identified listing, can be treated according to the parameters identified in **Table 6** below. The minimum treatment values and maximum treatment values are based on severity of the fungal infection. Severity of the infection is determined by the surface area, depth, colony count and symptoms. Application of therapy increases in intensity, duration and frequency as the severity increases. Minimum treatment parameters define the requirements for the initiation of the decolonization process.

### Example 6 - Table 6

| **Parameter** | **Minimum Value** | **Maximum Value** |
|---|---|---|
| Distance from exit to site (cm) | 1 | 50 |
| Time of Application (sec/cm²) | 5 | 90 |
| Number of treatments | 1 | 60 |
| Length of time between treatments (hours) | 3 | 168 |
| Temperature of plasma stream at contact with Site °C | 10 | 60 |
| Velocity of Plasma Stream at contact with Site (m/sec) | 0.05 | 200 |

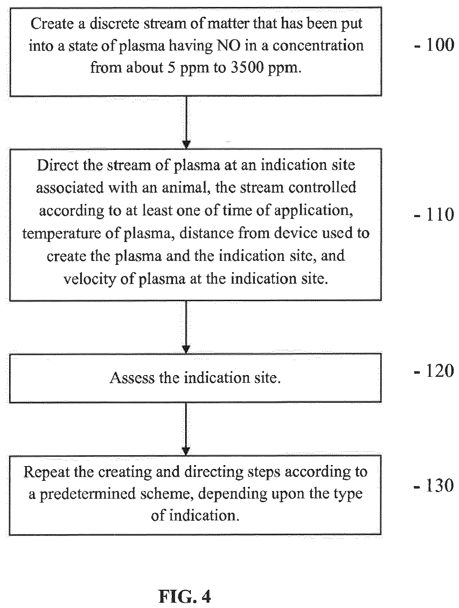

Referring now to **FIG. 4****,** a flow diagram illustrating an exemplary method for administering NO in a plasma state to a treatment site in accordance with the present disclosure is shown. At a first step 100 of the exemplary method, a discrete stream of matter that has been put into a state of plasma may be created, in which the stream has, as part of its content, NO in a concentration from about 5 ppm to 3500 ppm. At step 110, the stream of matter in a plasma state is directed at an indication site associated with an animal, where the stream is controlled according to at least one of time of application, temperature of the matter in a plasma state, distance from device used to create the matter in a plasma state and the indication site, and velocity of matter in a plasma state at the indication site. At step 120, the indication site is assessed. At step 130, the creating and directing steps are repeated according to a predetermined scheme, depending upon the type of indication.

As used herein, an element or step recited in the singular and proceeded with the word "a" or "an" should be understood as not excluding plural elements or steps, unless such exclusion is explicitly recited. Furthermore, references to "one embodiment" of the present invention are not intended to be interpreted as excluding the existence of additional embodiments that also incorporate the recited features. While the present invention has been disclosed with reference to certain embodiments, numerous modifications, alterations and changes to the described embodiments are possible without departing from the sphere and scope of the present invention, as defined in the appended claim(s). Accordingly, it is intended that the present invention not be limited to the described embodiments, but that it has the full scope defined by the language of the following claims, and equivalents thereof.

## Claims

1. A method for disinfecting clothes, shoes or equipment using nitric oxide, the method comprising:
creating a discrete stream of matter that includes nitric oxide (NO) in a concentration from about 5 ppm to 3500 ppm; and
directing the discrete stream of matter at a surface to disinfect the surface.

2. The method of claim 1, wherein the discrete stream of matter is in a state of plasma.

3. The method of claim 1, wherein the discrete stream of matter comprises:
| **Parameter** | **Minimum Value** | **Maximum Value** |
|---|---|---|
| Nitric Oxide (NO) | 5 ppm | 3500 ppm |
| NO₂ | 0 ppm | 200 ppm |
| N₂ | 75 vol % | 78 vol % |
| O₂ | 18 vol % | 21 vol % |
| O₃ | 0 ppm | 0.1 ppm |
| H₂O₂ | 0 ppm | 1500 ppm |
| H₂O | 0 ppm | 20000 ppm |
| Ar | 0 ppm | 9.1 * 10⁵ ppm |
| He | 5.2 ppm | 9.1 * 10⁵ ppm |
| CO | 0 ppm | 50 ppm |
| CO₂ | 0 ppm | 500 ppm |
| H₂ | 0 ppm | 10000 ppm |

4. The method of claim 1, wherein the temperature of the stream at contact with the site is from 10 to 60 degrees C.

5. The method of claim 1, wherein the velocity of the stream at contact with the site is from 0.05 to 200 meters per second.

6. The method of claim 1, comprising creating the discrete stream of matter using an electric arc discharge device.

7. The method of claim 1, comprising creating the discrete stream of matter using a microwave discharge device.

8. The method of claim 1, comprising creating the discrete stream of matter using a gliding arc discharge device.

9. The method of claim 1, wherein the equipment is veterinary equipment.
